(19) Europäisches Patentamt
European Patent Office
Office européen des brevets

(11) **EP 3 489 346 A1**

(12) **EUROPEAN PATENT APPLICATION**
published in accordance with Art. 153(4) EPC

(43) Date of publication:
**29.05.2019 Bulletin 2019/22**

(51) Int Cl.:
**C12M 3/00** (2006.01)          **C12M 1/00** (2006.01)
**C12N 1/00** (2006.01)          **C12N 5/00** (2006.01)

(21) Application number: **17834365.3**

(22) Date of filing: **25.07.2017**

(86) International application number:
**PCT/JP2017/026943**

(87) International publication number:
**WO 2018/021363 (01.02.2018 Gazette 2018/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **25.07.2016 JP 2016145815**

(71) Applicant: UBE Industries, Ltd.
Yamaguchi 755-8633 (JP)

(72) Inventors:
• **HAGIHARA, Masahiko**
**Ube-shi**
**Yamaguchi 755-8633 (JP)**

• **FUSE, Shinsaku**
**Ube-shi**
**Yamaguchi 755-8633 (JP)**
• **SHIMIZU, Motohisa**
**Ube-shi**
**Yamaguchi 755-8633 (JP)**
• **WADA, Yukinori**
**Ube-shi**
**Yamaguchi 755-8633 (JP)**

(74) Representative: **Plougmann Vingtoft a/s**
**Strandvejen 70**
**2900 Hellerup (DK)**

(54) **CELL CULTIVATION DEVICE AND CELL CULTIVATION METHOD USING SAME**

(57)     The present invention provides a cell cultivation device that is characterized by comprising: a polymer porous film; a cell cultivation part that has the polymer porous film; a shaft that passes through the cell cultivation part; and a cultivation tank in which at least one portion of the cell cultivation part is immersed, wherein the polymer porous film is a polymer porous film with a three-layer structure, having a surface layer A and a surface layer B that have a plurality of holes, and a macrovoid layer that is sandwiched between the surface layer A and the surface layer B, the average hole diameter of the holes present in the surface layer A is smaller than the average hole diameter of the holes present in the surface layer B, the macrovoid layer has dividing walls that are connected to the surface layers A and B, and a plurality of macrovoids that are surrounded by the dividing walls and the surface layers A and B, the holes in the surface layers A and B are in communication with the macrovoids, the cell cultivation part rotates with the shaft as the center, and the cells are alternately cultivated in a gas phase and a liquid phase.

FIG. 1

**Description**

FIELD

The present invention relates to a cell culture apparatus provided with a porous polymer film. In addition, it relates to a cell culture method using a cell culture apparatus provided with a porous polymer film.

BACKGROUND

In recent years, proteins such as enzymes, hormones, antibodies, cytokines, viruses (viral proteins) used for treatment and vaccine are industrially produced using cultured cells. However, such a protein production technology is expensive, raising medical cost. Accordingly, there have been demands for innovating technologies for culturing cells at high density and for increasing protein production, aiming at great reduction of cost.

As cells for protein production, anchorage-dependent adherent cells which adhere to a culture substrate may be sometimes used. Since such cells grow anchorage-dependently, they need to be cultured while being adhered onto the surface of a dish, plate or chamber. Conventionally, in order to culture such adherent cells in a large amount, it was preferable to increase the surface area to be adhered. However, increasing the culturing area inevitably requires to increase the space, which is responsible for increase in cost.

As a method to culture a large amount of adherent cells while decreasing the culture space, a culture method using a carrier having micropores, especially a microcarrier, has been developed (for example, PTL 1). In a cell culturing system using microcarriers, it is preferable to carry out sufficient stirring and diffusion so that the microcarriers do not aggregate together. Since this requires a volume allowing adequate agitation and diffusion of the medium in which the microcarriers are dispersed, there is an upper limit to the density at which the cells can be cultured. In order to separate the microcarrier from the medium, separation is preferably performed using a filter which can separate fine particles, possibly resulting in increased cost. Considering the foregoing, there is a demand for innovative methodology for cell culture which cultures cells at high density.

<Porous polyimide film>

Porous polyimide films have been utilized in the prior art for filters and low permittivity films, and especially for battery-related purposes, such as fuel cell electrolyte membrane and the like. PTLs 2 to 4 describe porous polyimide films with numerous macrovoids, having excellent permeability to objects such as gases, high porosity, excellent smoothness on both surfaces, relatively high strength and, despite high porosity, excellent resistance against compression stress in the film thickness direction. All of these are porous polyimide films formed via amic acid.

The cell culture method which includes applying cells to a porous polyimide film and culturing them is reported (PTL 5).

[CITATION LIST]

[PATENT LITERATURE]

[PTL 1] WO2003/054174
[PTL 2] WO2010/038873
[PTL 3] Japanese Unexamined Patent Publication (Kokai) No. 2011-219585
[PTL 4] Japanese Unexamined Patent Publication (Kokai) No. 2011-219586
[PTL 5] WO2015/012415

SUMMARY

[TECHNICAL PROBLEM]

The present invention aims at providing a cell culture apparatus provided with a porous polymer film. In addition, the present invention also aims at providing a cell culture method using a cell culture apparatus provided with a porous polymer film.

[SOLUTION TO PROBLEM]

The present inventors have found that a porous polymer film having a predetermined structure provides not only an optimum space capable of culturing a large amount of cells but also a wet environment resistant to drying. Accordingly, we have completed an apparatus for supporting and culturing cells on a porous polymer film while exposing them to a gas phase, and a culture method using such an apparatus. In other words, the present invention preferably includes, but is not limited to, the following modes.

[1] A cell culture apparatus, the apparatus characterized by comprising:

a porous polymer film;
a cell culture section having the porous polymer film;
a shaft penetrating the cell culture section; and
a medium tank immersing at least a part of the cell culture section;

wherein the porous polymer films are a three-layer structure porous polymer film having a surface layer A and a surface layer B, the surface layers having a plurality of pores, and a macrovoid layer sandwiched between the surface layers A and B;
wherein an average pore diameter of the pores present in the surface layer A is smaller than an average pore diameter of the pores present in the surface layer B;
wherein the macrovoid layer has a partition wall bonded to the surface layers A and B, and a plurality of macrovoids surrounded by such a partition wall and the surface layers A and B;
wherein the pores in the surface layers A and B communicate with the macrovoid; and
wherein the cell culture section rotates around the shaft, and cells carried on the porous polymer films are alternately cultured in a gas phase and a liquid phase.
[2] The cell culture apparatus according to [1], the apparatus further comprising a rotary motor for rotating the shaft.
[3] The cell culture apparatus according to [1] or [2], wherein the cell culture section comprises a porous polymer film support, and a porous polymer film clamp,
wherein the porous polymer film is sandwiched between the porous polymer film support and the porous polymer film clamp.
[4] The cell culture apparatus according to [3], wherein two or more sheets of the porous polymer films are stacked and sandwiched.
[5] The cell culture apparatus according to [1] or [2], wherein the cell culture section is a cylindrical container;
wherein the end face of the cylindrical container comprises one or more medium flow inlets;
wherein the side face of the cylindrical container comprises one or more medium flow inlets; and
wherein the porous polymer film is contained in the cylindrical containers.
[6] The cell culture apparatus according to [5], wherein the cylindrical container comprises one or more helical flow paths that communicate a part of one end face of the cylindrical container with a part of the other end face.
[7] The cell culture apparatus according to [5] or [6], wherein the porous polymer film is contained in the cylindrical container, with the porous polymer film:

i) being folded up;
ii) being wound into a roll-like shape;
iii) sheets or pieces thereof being concatenated with a thread-like structure;
iv) being tied together into a rope-like shape; and/or
v) two or more thereof being stacked.

[8] The cell culture apparatus according to [5] or [6], wherein the porous polymer film is a modularized porous polymer film having a casing;
wherein the modularized porous polymer film is contained within the casing with:

(i) the two or more independent porous polymer films being aggregated;
(ii) the porous polymer film being folded up;
(iii) the porous polymer film being wound into a roll-like shape; and/or
(iv) the porous polymer film being tied together into a rope-like shape; wherein the modularized porous polymer film is contained in the cylindrical container.

[9] The cell culture apparatus according to any one of [1] to [8], wherein two or more of the cell culture sections have been concatenated.

[10] The cell culture apparatus according to any one of [1] to [9], the apparatus further comprising:

a medium discharging line communicating with the medium tank at one end;
a medium discharging tank communicating with the other end of the medium discharging line;
a medium supplying line communicating at one end with the culture medium discharging tank; and
a medium supplying pump provided in the middle of the medium supplying line.

[11] The cell culture apparatus according to any one of [1] to [10], characterized by further comprising medium droplet supply means, wherein a dropletized medium is supplied to the porous polymer film.

[12] The cell culture apparatus according to any one of [1] to [11], wherein the porous polymer film has a plurality of pores having an average pore diameter of 0.01 to 100 $\mu$m.

[13] The cell culture apparatus according to any one of [1] to [12], wherein an average pore diameter of the surface layer A is 0.01 to 50 $\mu$m.

[14] The cell culture apparatus according to any one of [1] to [13], wherein an average pore diameter of the surface layer B is 20 to 100 $\mu$m.

[15] The cell culture apparatus according to any one of [1] to [14], wherein a total film thickness of the porous polymer film is 5 to 500 $\mu$m.

[16] The cell culture apparatus according to any one of [1] to [15], wherein the porous polymer film is a porous polyimide film.

[17] The cell culture apparatus according to [16], wherein the porous polyimide film is a porous polyimide film comprising a polyimide derived from tetracarboxylic dianhydride and diamine.

[18] The cell culture apparatus according to [16] or [17], wherein the porous polyimide film is a colored porous polyimide film that is obtained by molding a polyamic acid solution composition comprising a polyamic acid solution derived from tetracarboxylic dianhydride and diamine, and a coloring precursor, and subsequently heat-treating the resultant composition at 250°C or higher.

[19] The cell culture apparatus according to any one of [1] to [15], wherein the porous polymer film is a porous polyethersulfone film.

[20] A culture method using the cell culture apparatus according to any one of [1] to [19], the method characterized by comprising:
rotating the cell culture section around the shaft, and alternately culturing the cell carried on the porous polymer film in a gas phase and a liquid phase.

[ADVANTAGEOUS EFFECTS OF INVENTION]

By using a porous polymer film as a cell culture support, the present invention allows cell culturing to be carried out conveniently, efficiently and continuously, even under conditions with a small culturing space and low medium volume. Since a porous polymer film has a low hydrophilic porous property, liquid is stably held in the porous polymer film, and a wet environment is maintained that is resistant to drying. It is therefore possible to achieve survival and proliferation of cells even in very small amounts of medium, even when compared with conventional cell culturing apparatuses. Furthermore, since it is possible to carry out culturing even if all or some of the porous polymer film has been exposed to air, oxygen can be efficiently supplied to the cells, and mass culturing of cells is made possible.

According to the invention, the amount of medium used is extremely minimal, and the porous polymer film used as the culture support can be exposed to a gas phase, thereby allowing oxygen supply to the cells to be adequately accomplished by repeated diffusion to the gas phase. According to the invention, therefore, there is no particular need for an oxygen supply apparatus.

BRIEF DESCRIPTION OF DRAWINGS

FIG. 1 represents a perspective view illustrating a configurational example of a cell culture apparatus in an embodiment.
FIG. 2 is a perspective view illustrating a configurational example of a cell culture apparatus according to an embodiment of the invention.
FIG. 3 represents a diagram illustrating a culture tank in an embodiment. (A) represents a planar view, (B) represents a left side view, (C) represents a cross sectional view taken along Z-Z' line in (A), and (C) represents a right side view.
FIG. 4 represents a diagram illustrating a cell culture section in an embodiment. (A) represents a porous polymer film support (left panel, a planar view; right panel, a cross sectional view); and (B) represents a porous polymer film clamp (left panel, a planar view; right panel, a cross sectional view).

FIG. 5 represents a perspective view illustrating a cell culture section (cylindrical container) in an embodiment. Top and bottom views represent the same cylindrical container as viewed from different angles.

FIG. 6 is a model diagram of cell culturing using a porous polymer film.

FIG. 7 is a diagram illustrating a cell culture apparatus according to an embodiment of the invention.

FIG. 8 is a diagram illustrating a cell culture apparatus according to an embodiment of the invention.

FIG. 9 is a diagram illustrating a cell culture apparatus according to an embodiment of the invention. (A) represents a module, (B) represents a cell culture section, and (C) represents a cell culture apparatus in an embodiment.

DESCRIPTION OF EMBODIMENTS

The embodiments of the present invention will be hereinafter described with reference to drawings as needed. The configuration of the embodiments is illustrative, and the configuration of the present invention is not limited to the specific configuration of the embodiment.

1. Porous polymer film

The average pore diameter of the pore present on a surface layer A (hereinafter referred to as "surface A" or "mesh surface") in the porous polymer film used for the present invention is not particularly limited, but is, for example, 0.01 $\mu$m or more and less than 200 $\mu$m, 0.01 to 150 $\mu$m, 0.01 to 100 $\mu$m, 0.01 to 50 $\mu$m, 0.01 to 40 $\mu$m, 0.01 to 30 $\mu$m, 0.01 to 20 $\mu$m, or 0.01 to 15 $\mu$m, preferably 0.01 to 15 $\mu$m.

The average pore diameter of the pore present on a surface layer B (hereinafter referred to as "surface B" or "large pore surface") in the porous polymer film used for the present invention is not particularly limited so long as it is larger than the average pore diameter of the pore present on the surface A, but is, for example, greater than 5 $\mu$m and 200 $\mu$m or less, 20 $\mu$m to 100 $\mu$m, 30 $\mu$m to 100 $\mu$m, 40 $\mu$m to 100 $\mu$m, 50 $\mu$m to 100 $\mu$m, or 60 $\mu$m to 100 $\mu$m, preferably 20 $\mu$m to 100 $\mu$m.

The average pore diameter on the surface of the porous polymer film is determined by measuring pore area for 200 or more open pore portions, and calculated an average diameter according to the following Equation (1) from the average pore area assuming the pore shape as a perfect circle.

[Math. 1]

$$\text{Average Pore Size} = 2 \times \sqrt{(S_a / \pi)} \qquad (1)$$

(wherein Sa represents the average value for the pore areas).

The thicknesses of the surface layers A and B are not particularly limited, but are, for example, 0.01 to 50 $\mu$m, preferably 0.01 to 20 $\mu$m.

The average pore diameter of macrovoids in the planar direction of the film in the macrovoid layer in the porous polymer film is not particularly limited but is, for example, 10 to 500 $\mu$m, preferably 10 to 100 $\mu$m, and more preferably 10 to 80 $\mu$m. The thicknesses of the partition wall in the macrovoid layer are not particularly limited, but are, for example, 0.01 to 50 $\mu$m, preferably 0.01 to 20 $\mu$m. In an embodiment, at least one partition wall in the macrovoid layer has one or two or more pores connecting the neighboring macrovoids and having the average pore diameter of 0.01 to 100 $\mu$m, preferably 0.01 to 50 $\mu$m. In another embodiment, the partition wall in the macrovoid layer has no pore.

The total film thickness of the porous polymer film used for the invention is not particularly limited, but may be 5 $\mu$m or more, 10 $\mu$m or more, 20 $\mu$m or more or 25 $\mu$m or more, and 500 $\mu$m or less, 300 $\mu$m or less, 100 $\mu$m or less, 75 $\mu$m or less, or 50 $\mu$m or less. It is preferably 5 to 500 $\mu$m, and more preferably 25 to 75 $\mu$m.

The film thickness of the porous polymer film used for the invention can be measured using a contact thickness gauge.

The porosity of the porous polymer film used in the present invention is not particularly limited but is, for example, 40% or more and less than 95%.

The porosity of the porous polymer film used for the invention can be determined by measuring the film thickness and mass of the porous film cut out to a prescribed size, and performing calculation from the basis weight according to the following Equation (2).

[Math. 2]

$$\text{Porosity (\%)} = (1 - w/(S \times d \times D)) \times 100 \qquad (2)$$

(wherein S represents the area of the porous film, d represents the total film thickness, w represents the measured mass, and D represents the polymer density. The density is defined as 1.34 g/cm$^3$ when the polymer is a polyimide.)

The porous polymer film used for the present invention is preferably a porous polymer film which includes a three-layer structure porous polymer film having a surface layer A and a surface layer B, the surface layers having a plurality of pores, and a macrovoid layer sandwiched between the surface layers A and B; wherein the average pore diameter of the pore present on the surface layer A is 0.01 μm to 15 μm, and the average pore diameter of the pore present on the surface layer B is 20 μm to 100 μm; wherein the macrovoid layer has a partition wall bonded to the surface layers A and B, and a plurality of macrovoids surrounded by such a partition wall and the surface layers A and B, the thickness of the macrovoid layer, and the surface layers A and B is 0.01 to 20 μm; wherein the pores on the surface layers A and B communicate with the macrovoid, the total film thickness is 5 to 500 μm, and the porosity is 40% or more and less than 95%. In an embodiment, at least one partition wall in the macrovoid layer has one or two or more pores communicating the neighboring macrovoids with each other and having the average pore diameter of 0.01 to 100 μm, preferably 0.01 to 50 μm. In another embodiment, the partition wall has does not have such pores.

The porous polymer film used in the invention is preferably sterilized. The sterilization treatment is not particularly limited, but any sterilization treatment such as dry heat sterilization, steam sterilization, sterilization with a disinfectant such as ethanol, electromagnetic wave sterilization such as ultraviolet rays or gamma rays, and the like can be mentioned.

The porous polymer film used for the present invention has the structural features described above and is not particularly limited but includes, preferably a porous polyimide film or porous polyethersulfone film.

1-1. Porous polyimide film

Polyimide is a general term for polymers containing imide bonds in the repeating unit, and usually it refers to an aromatic polyimide in which aromatic compounds are directly linked by imide bonds. An aromatic polyimide has an aromatic-aromatic conjugated structure via an imide bond, and therefore has a strong rigid molecular structure, and since the imide bonds provide powerful intermolecular force, it has very high levels of thermal, mechanical and chemical properties.

The porous polyimide film which can be used for the invention is preferably a porous polyimide film including (as a main component) a polyimide obtained from a tetracarboxylic dianhydride and a diamine, and more preferably, it is a porous polyimide film comprising a polyimide obtained from a tetracarboxylic dianhydride and a diamine. The phrase "including as the main component" means that it essentially contains no components other than the polyimide obtained from a tetracarboxylic dianhydride and a diamine, as constituent components of the porous polyimide film, or that it may contain them but they are additional components that do not affect the properties of the polyimide obtained from the tetracarboxylic dianhydride and diamine.

In an embodiment, the porous polyimide film which may be used for the present invention also includes colored porous polyimide films obtained by forming a polyamic acid solution composition containing a polyamic acid solution obtained from a tetracarboxylic acid component and a diamine component, and a coloring precursor, and then heat treating it at 250°C or higher.

A polyamic acid is obtained by polymerization of a tetracarboxylic acid component and a diamine component. A polyamic acid is a polyimide precursor that can be cyclized to a polyimide by thermal imidization or chemical imidization.

The polyamic acid used may be any one that does not have an effect on the invention, even if a portion of the amic acid is imidized. Specifically, the polyamic acid may be partially thermally imidized or chemically imidized.

When the polyamic acid is to be thermally imidized, there may be added to the polyamic acid solution, if necessary, an imidization catalyst, an organic phosphorus-containing compound, or fine particles such as inorganic fine particles or organic fine particles. Further, when the polyamic acid is to be chemically imidized, there may be added to the polyamic acid solution, if necessary, a chemical imidization agent, a dehydrating agent, or fine particles such as inorganic fine particles or organic fine particles. Even if such components are added to the polyamic acid solution, they are preferably added under conditions that do not cause precipitation of the coloring precursor.

In this specification, a "coloring precursor" is a precursor that generates a colored substance by partial or total carbonization under heat treatment at 250°C or higher.

Coloring precursors usable for the production of the porous polyimide film are preferably uniformly dissolved or dispersed in a polyamic acid solution or polyimide solution and subjected to thermal decomposition by heat treatment at 250°C or higher, preferably 260°C or higher, even more preferably 280°C or higher and more preferably 300°C or higher, and preferably heat treatment in the presence of oxygen such as air, at 250°C or higher, preferably 260°C or higher, even more preferably 280°C or higher and more preferably 300°C or higher, for carbonization to produce a colored substance, more preferably producing a black colored substance, with carbon-based coloring precursors being more preferred.

The coloring precursor, when being heated, first appears as a carbonized compound, but compositionally it contains other elements in addition to carbon, and also includes layered structures, aromatic crosslinked structures and tetrahedron carbon-containing disordered structures.

Carbon-based coloring precursors are not particularly restricted, and for example, they include tar or pitch such as petroleum tar, petroleum pitch, coal tar and coal pitch, coke, polymers obtained from acrylonitrile-containing monomers, ferrocene compounds (ferrocene and ferrocene derivatives), and the like. Of these, polymers obtained from acrylonitrile-containing monomers and/or ferrocene compounds are preferred, with polyacrylonitrile being preferred as a polymer obtained from an acrylonitrile-containing monomer.

Moreover, in another embodiment, examples of the porous polyimide film which may be used for the preset invention also include a porous polyimide film which can be obtained by molding a polyamic acid solution derived from a tetracarboxylic acid component and a diamine component followed by heat treatment without using the coloring precursor.

The porous polyimide film produced without using the coloring precursor may be produced, for example, by casting a polyamic acid solution into a film, the polyamic acid solution being composed of 3 to 60% by mass of polyamic acid having an intrinsic viscosity number of 1.0 to 3.0 and 40 to 97% by mass of an organic polar solvent, immersing or contacting in a coagulating solvent containing water as an essential component, and imidating the porous film of the polyamic acid by heat treatment. In this method, the coagulating solvent containing water as an essential component may be water, or a mixed solution containing 5% by mass or more and less than 100% by mass of water and more than 0% by mass and 95% by mass or less of an organic polar solvent. Further, after the imidation, at least one surface of the resulting porous polyimide film may be subjected to plasma treatment.

The tetracarboxylic dianhydride which may be used for the production of the porous polyimide film may be any tetracarboxylic dianhydride, selected as appropriate according to the properties desired. Specific examples of tetracarboxylic dianhydrides include biphenyltetracarboxylic dianhydrides such as pyromellitic dianhydride, 3,3',4,4'-biphenyltetracarboxylic dianhydride (s-BPDA) and 2,3,3',4'-biphenyltetracarboxylic dianhydride (a-BPDA), oxydiphthalic dianhydride, diphenylsulfone-3,4,3',4'-tetracarboxylic dianhydride, bis(3,4-dicarboxyphenyl)sulfide dianhydride, 2,2-bis(3,4-dicarboxyphenyl)-1,1,1,3,3,3-hexafluoropropane dianhydride, 2,3,3',4'-benzophenonetetracarboxylic dianhydride, 3,3',4,4'-benzophenonetetracarboxylic dianhydride, bis(3,4-dicarboxyphenyl)methane dianhydride, 2,2-bis(3,4-dicarboxyphenyl)propane dianhydride, p-phenylenebis(trimellitic acid monoester acid anhydride), p-biphenylenebis(trimellitic acid monoester acid anhydride), m-terphenyl-3,4,3',4'-tetracarboxylic dianhydride, p-terphenyl-3,4,3',4'-tetracarboxylic dianhydride, 1,3-bis(3,4-dicarboxyphenoxy)benzene dianhydride, 1,4-bis(3,4-dicarboxyphenoxy)benzene dianhydride, 1,4-bis(3,4-dicarboxyphenoxy)biphenyl dianhydride, 2,2-bis[(3,4-dicarboxyphenoxy)phenyl]propane dianhydride, 2,3,6,7-naphthalenetetracarboxylic dianhydride, 1,4,5,8-naphthalenetetracarboxylic dianhydride, 4,4'-(2,2-hexafluoroisopropylidene)diphthalic dianhydride, and the like. Further preferably used is an aromatic tetracarboxylic acid such as 2,3,3',4'-diphenylsulfonetetracarboxylic acid. These may be used alone or in appropriate combinations of two or more.

Particularly preferred among these are at least one type of aromatic tetracarboxylic dianhydride selected from the group consisting of biphenyltetracarboxylic dianhydride and pyromellitic dianhydride. As a biphenyltetracarboxylic dianhydride there may be suitably used 3,3',4,4'-biphenyltetracarboxylic dianhydride.

As diamine which may be used for the production of the porous polyimide film, any diamine may be used. Specific examples of diamines include the following:

1) Benzenediamines with one benzene nucleus, such as 1,4-diaminobenzene(paraphenylenediamine), 1,3-diaminobenzene, 2,4-diaminotoluene and 2,6-diaminotoluene;

2) diamines with two benzene nuclei, including diaminodiphenyl ethers such as 4,4'-diaminodiphenyl ether and 3,4'-diaminodiphenyl ether, and 4,4'-diaminodiphenylmethane, 3,3'-dimethyl-4,4'-diaminobiphenyl, 2,2'-dimethyl-4,4'-diaminobiphenyl, 2,2'-bis(trifluoromethyl)-4,4'-diaminobiphenyl, 3,3'-dimethyl-4,4'-diaminodiphenylmethane, 3,3'-dicarboxy-4,4'-diaminodiphenylmethane, 3,3',5,5'-tetramethyl-4,4'-diaminodiphenylmethane, bis(4-aminophenyl)sulfide, 4,4'-diaminobenzanilide, 3,3'-dichlorobenzidine, 3,3'-dimethylbenzidine, 2,2'-dimethylbenzidine, 3,3'-dimethoxybenzidine, 2,2'-dimethoxybenzidine, 3,3'-diaminodiphenyl ether, 3,4'-diaminodiphenyl ether, 4,4'-diaminodiphenyl ether, 3,3'-diaminodiphenyl sulfide, 3,4'-diaminodiphenyl sulfide, 4,4'-diaminodiphenyl sulfide, 3,3'-diaminodiphenylsulfone, 3,4'-diaminodiphenylsulfone, 4,4'-diaminodiphenylsulfone, 3,3'-diaminobenzophenone, 3,3'-diamino-4,4'-dichlorobenzophenone, 3,3'-diamino-4,4'-dimethoxybenzophenone, 3,3'-diaminodiphenylmethane, 3,4'-diaminodiphenylmethane, 4,4'-diaminodiphenylmethane, 2,2-bis(3-aminophenyl)propane, 2,2-bis(4-aminophenyl)propane, 2,2-bis(3-aminophenyl)-1,1,1,3,3,3-hexafluoropropane, 2,2-bis(4-aminophenyl)-1,1,1,3,3,3-hexafluoropropane, 3,3'-diaminodiphenyl sulfoxide, 3,4'-diaminodiphenyl sulfoxide and 4,4'-diaminodiphenyl sulfoxide;

3) diamines with three benzene nuclei, including 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene, 1,4-bis(3-aminophenoxy)benzene, 1,4-bis(4-aminophenoxy)benzene, 1,3-bis(3-aminophenoxy)-4-trifluoromethylbenzene, 3,3'-diamino-4-(4-phenyl)phenoxybenzophenone, 3,3'-diamino-4,4'-di-(4-phenylphenoxy)benzophenone, 1,3-bis(3-aminophenyl sulfide)benzene, 1,3-bis(4-aminophenyl sulfide)benzene, 1,4-bis(4-aminophenyl sulfide)benzene, 1,3-bis(3-aminophenylsulfone)benzene, 1,3-bis(4-aminophenylsulfone)benzene, 1,4-bis(4-aminophenylsulfone)benzene, 1,3-bis[2-(4-aminophenyl)isopropyl]benzene, 1,4-bis[2-(3-aminophenyl)isopropyl]benzene and 1,4-bis[2-(4-aminophenyl)isopropyl]benzene;

4) diamines with four benzene nuclei, including 3,3'-bis(3-aminophenoxy)biphenyl, 3,3'-bis(4-aminophenoxy)biphenyl, 4,4'-bis(3-aminophenoxy)biphenyl, 4,4'-bis(4-aminophenoxy)biphenyl, bis[3-(3-aminophenoxy)phenyl] ether, bis[3-(4-aminophenoxy)phenyl] ether, bis[4-(3-aminophenoxy)phenyl] ether, bis [4-(4-aminophenoxy)phenyl]ether, bis[3-(3-aminophenoxy)phenyl]ketone, bis[3-(4-aminophenoxy)phenyl]ketone, bis[4-(3-aminophenoxy)phenyl]ketone, bis[4-(4-aminophenoxy)phenyl]ketone, bis[3-(3-aminophenoxy)phenyl] sulfide, bis[3-(4-aminophenoxy)phenyl] sulfide, bis[4-(3-aminophenoxy)phenyl] sulfide, bis[4-(4-aminophenoxy)phenyl] sulfide, bis[3-(3-aminophenoxy)phenyl]sulfone, bis[3-(4-aminophenoxy)phenyl]sulfone, bis[4-(3-aminophenoxy)phenyl]sulfone, bis[4-(4-aminophenoxy)phenyl]sulfone, bis[3-(3-aminophenoxy)phenyl]methane, bis[3-(4-aminophenoxy)phenyl]methane, bis[4-(3-aminophenoxy)phenyl]methane, bis [4-(4-aminophenoxy)phenyl]methane, 2,2-bis[3-(3-aminophenoxy)phenyl]propane, 2,2-bis[3-(4-aminophenoxy)phenyl]propane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[3-(4-aminophenoxy)phenyl]-1,1,1,3,3,3-hexafluoropropane, 2,2-bis[4-(3-aminophenoxy)phenyl]-1,1,1,3,3,3-hexafluoropropane and 2,2-bis[4-(4-aminophenoxy)phenyl]-1,1,1,3,3,3-hexafluoropropane.

These may be used alone or in mixtures of two or more. The diamine used may be appropriately selected according to the properties desired.

Preferred among these are aromatic diamine compounds, with 3,3'-diaminodiphenyl ether, 3,4'-diaminodiphenyl ether, 4,4'-diaminodiphenyl ether, paraphenylenediamine, 1,3-bis(3-aminophenyl)benzene, 1,3-bis(4-aminophenyl)benzene, 1,4-bis(3-aminophenyl)benzene, 1,4-bis(4-aminophenyl)benzene, 1,3-bis(4-aminophenoxy)benzene and 1,4-bis(3-aminophenoxy)benzene being preferred for use. Particularly, at least one type of diamine selected from the group consisting of benzenediamines, diaminodiphenyl ethers and bis(aminophenoxy)phenyl is preferred.

From the viewpoint of heat resistance and dimensional stability under high temperature, the porous polyimide film which may be used for the invention is preferably formed from a polyimide obtained by combination of a tetracarboxylic dianhydride and a diamine, having a glass transition temperature of 240°C or higher, or without a distinct transition point at 300°C or higher.

From the viewpoint of heat resistance and dimensional stability under high temperature, the porous polyimide film which can be used for the invention is preferably a porous polyimide film comprising one of the following aromatic polyimides.

(i) an aromatic polyimide comprising at least one tetracarboxylic acid unit selected from the group consisting of biphenyltetracarboxylic acid units and pyromellitic acid units, and an aromatic diamine unit,
(ii) an aromatic polyimide comprising a tetracarboxylic acid unit and at least one type of aromatic diamine unit selected from the group consisting of benzenediamine units, diaminodiphenyl ether units and bis(aminophenoxy)phenyl units,
and/or,
(iii) an aromatic polyimide comprising at least one type of tetracarboxylic acid unit selected from the group consisting of biphenyltetracarboxylic acid units and pyromellitic acid units, and at least one type of aromatic diamine unit selected from the group consisting of benzenediamine units, diaminodiphenyl ether units and bis(aminophenoxy)phenyl units.

The porous polyimide film used for the present invention is preferably a porous polyimide film which includes a three-layer structure porous polyimide film having a surface layer A and a surface layer B, the surface layers having a plurality of pores, and a macrovoid layer sandwiched between the surface layers A and B; wherein the average pore diameter of the pore present on the surface layer A is 0.01 μm to 15 μm, and the average pore diameter of the pore present on the surface layer B is 20 μm to 100 μm; wherein the macrovoid layer has a partition wall bonded to the surface layers A and B, and a plurality of macrovoids surrounded by such a partition wall and the surface layers A and B, the thickness of the macrovoid layer, and the surface layers A and B is 0.01 to 20 μm; wherein the pores on the surface layers A and B communicate with the macrovoid, the total film thickness is 5 to 500 μm, and the porosity is 40% or more and less than 95%. In this case, at least one partition wall in the macrovoid layer has one or two or more pores connecting the neighboring macrovoids and having the average pore diameter of 0.01 to 100 μm, preferably 0.01 to 50 μm.

For example, porous polyimide films described in WO2010/038873, Japanese Unexamined Patent Publication No. 2011-219585 or Japanese Unexamined Patent Publication No. 2011-219586 can be used for the present invention.

1-2. Porous polyethersulfone film (Porous PES film)

The porous polyethersulfone film which may be used for the present invention contains polyethersulfone and typically consists substantially of polyethersulfone. Polyethersulfone may be synthesized by the method known to those skilled in the art. For example, it may be produced by a method wherein a dihydric phenol, an alkaline metal compound and a dihalogenodiphenyl compound are subjected to polycondensation reaction in an organic polar solvent, a method wherein a previously synthesized alkaline metal di-salt of a dihydric phenol previously synthesized is subjected to polycondensation reaction dihalogenodiphenyl compound in an organic polar solvent or the like.

Examples of an alkaline metal compound include alkaline metal carbonate, alkaline metal hydroxide, alkaline metal hydride, alkaline metal alkoxide and the like. Particularly, sodium carbonate and potassium carbonate are preferred.

Examples of a dihydric phenol compound include hydroquinone, catechol, resorcin, 4,4'-biphenol, bis (hydroxyphenyl)alkanes (such as 2,2-bis(hydroxyphenyl)propane, and 2,2-bis(hydroxyphenyl)methane), dihydroxydiphenylsulfones, dihydroxydiphenyl ethers, or those mentioned above having at least one hydrogen on the benzene rings thereof substituted with a lower alkyl group such as a methyl group, an ethyl group, or a propyl group, or with a lower alkoxy group such as a methoxy group, or an ethoxy group. As the dihydric phenol compound, two or more of the aforementioned compounds may be mixed and used.

Polyethersulfone may be a commercially available product. Examples of a commercially available product include SUMIKAEXCEL 7600P, SUMIKAEXCEL 5900P (both manufactured by Sumitomo Chemical Company, Limited).

The logarithmic viscosity of the polyethersulfone is preferably 0.5 or more, more preferably 0.55 or more from the viewpoint of favorable formation of a macrovoid of the porous polyethersulfone film; and it is preferably 1.0 or less, more preferably 0.9 or less, further preferably 0.8 or less, particularly preferably 0.75 or less from the viewpoint of the easy production of a porous polyethersulfone film.

Further, from the viewpoints of heat resistance and dimensional stability under high temperature, it is preferred that the porous polyethersulfone film or polyethersulfone as a raw material thereof has a glass transition temperature of 200°C or higher, or that a distinct glass transition temperature is not observed.

The method for producing the porous polyethersulfone film which may be used for the present invention is not particularly limited. For example, the film may be produced by a method including the following steps:

a step in which polyethersulfone solution containing 0.3 to 60% by mass of polyethersulfone having logarithmic viscosity of 0.5 to 1.0 and 40 to 99.7% by mass of an organic polar solvent is casted into a film, immersed in or contacted with a coagulating solvent containing a poor solvent or non-solvent of polyethersulfone to produce a coagulated film having pores; and

a step in which the coagulated film having pores obtained in the above-mentioned step is heat-treated for coarsening of the aforementioned pores to obtain a porous polyethersulfone film;

wherein the heat treatment includes the temperature of the coagulated film having the pores is raised higher than the glass transition temperature of the polyethersulfone, or up to 240°C or higher.

The porous polyethersulfone film which can be used in the present invention is preferably a porous polyethersulfone film having a surface layer A, a surface layer B, and a macrovoid layer sandwiched between the surface layers A and B,

wherein the macrovoid layer has a partition wall bonded to the surface layers A and B, and a plurality of macrovoids surrounded by such a partition wall and the surface layers A and B, the macrovoids having the average pore diameter in the planar direction of the film of 10 to 500 $\mu$m;

wherein the thickness of the macrovoid layer is 0.1 to 50 $\mu$m,

each of the surface layers A and B has a thickness of 0.1 to 50 $\mu$m,

wherein one of the surface layers A and B has a plurality of pores having the average pore diameter of more than 5 $\mu$m and 200 $\mu$m or less, while the other has a plurality of pores having the average pore diameter of 0.01 $\mu$m or more and less than 200 $\mu$m,

wherein one of the surface layers A and B has a surface aperture ratio of 15% or more while other has a surface aperture ratio of 10% or more,

wherein the pores of the surface layers A and B communicate with the macrovoids,

wherein the porous polyethersulfone film has total film thickness of 5 to 500 $\mu$m and a porosity of 50 to 95%.

Since the aforementioned porous polymer film as a cell culture carrier which may be used for the cell culture apparatus of the present invention has a low hydrophilic porous property, liquid is stably held in the porous polymer film, and a wet environment is maintained that is also resistant to drying. It is therefore possible to achieve survival and proliferation of cells even in very small amounts of medium, even when compared with the cell culture apparatus utilizing the conventional cell culture carriers. Furthermore, since it is possible to carry out culturing even if some or all of the porous polymer film has been exposed to air, oxygen can be efficiently supplied to the cells, and mass culturing of cells is made possible.

According to the invention, the amount of medium used is extremely minimal, and the porous polymer film as the culture support can be exposed to a gas phase, thereby allowing oxygen supply to the cells to be adequately accomplished by diffusion. According to the invention, therefore, there is no particular need for an oxygen supply means.

2. Cell culture apparatus

An embodiment of the invention relates to a cell culture apparatus including:

a porous polymer film;
a cell culture section having a porous polymer film;
a shaft penetrating the cell culture section; and
a medium tank immersing at least a part of the cell culture section;
wherein the porous polymer film is a three-layer structure porous polymer film having a surface layer A and a surface layer B, the surface layers having a plurality of pores, and a macrovoid layer sandwiched between the surface layers A and B; wherein an average pore diameter of the pores present in the surface layer A is smaller than an average pore diameter of the pores present in the surface layer B; wherein the macrovoid layer has a partition wall bonded to the surface layers A and B, and a plurality of macrovoids surrounded by such a partition wall and the surface layers A and B;
wherein the cell culture section rotates around the shaft, and the cells carried on the porous polymer film are cultured alternately in a gas phase and a liquid phase. The cell culture apparatus will be hereinafter also referred to as a "cell culture apparatus of the present invention". The embodiment of the cell culture apparatus of the present invention will be illustrated with reference to the drawings.

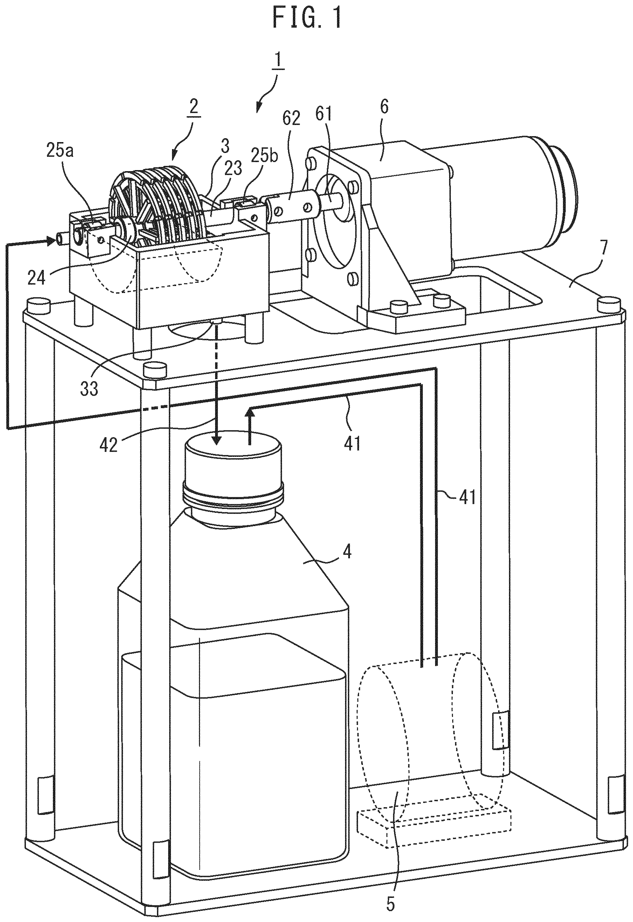

FIG. 1 is a diagram illustrating a configurational example of a cell culture apparatus 1 of the invention. The cell culture apparatus 1 includes a cell culture section 2 having the porous polymer film, a shaft 23 penetrating the center of the cell culture section 2, and a medium tank 3 in which at least a part of the cell culture section 2 is immersed. The medium tank 3 is filled with a predetermined amount of a medium. A part of the cell culture section 2 is periodically immersed in a medium by rotating the cell culture section 2 around the shaft 23. In this way, the cells carried on the porous polymer film is cultured alternately in gas phase and liquid phase. It is possible to conduct cell culture in a gas phase like in the present embodiment. It is because the porous polymer film used in the present invention may stably hold liquid and may provide a wet environment resistant to drying. Using a porous polymer film of the present invention makes it possible to realize the configuration of the present invention.

This embodiment further includes a rotary motor 6 fixed to a shaft 23 at a shaft joint 62. In this embodiment, the shaft 23 is fixed to a cell culture section 2 by an arbitrary means. In this way, the operation of the rotary motor 6 rotates a rotary motor shaft 61, the rotational motion is transmitted to the shaft 23. Thus, the cell culture section 2 fixed to the shaft 23 is rotated. The rotary motor 6 may be controlled by a computer or the like. For example, by combining rotating speed, initiation, termination, etc., rotation may be controlled under conditions optimum for cell culture.

This embodiment further includes a medium discharging line 42. The one end portion of the medium discharging line 42 is connected to a medium discharging pipe 33 of the medium tank 3 while the other end portion of the medium discharging line 42 is connected to the medium discharging tank 4. Accordingly, the medium discharged from the medium tank 3 is pooled in the medium discharging tank 4. This embodiment further includes a medium supplying line 41. The one end portion of the medium supplying line 41 is connected to the medium discharging tank 4, while the other end portion of the medium supplying line 41 is connected to the medium supplying pipe 34 of the medium tank 3. In addition, a medium supplying pump 5 is provided in the middle of the medium supplying line 41. The operation of the medium supplying pump 5 makes the medium in the medium discharging tank 4 to be transferred again to the medium tank 3. The types of the medium supplying pump 5 is not particularly limited. For example, a tube pump, a peristaltic pump may be used. In this embodiment, a part of the cell culture apparatus 1 is installed on an installation table 7. The installation table 7 may not be always used so long as the configuration may exhibit the effect of the present invention.

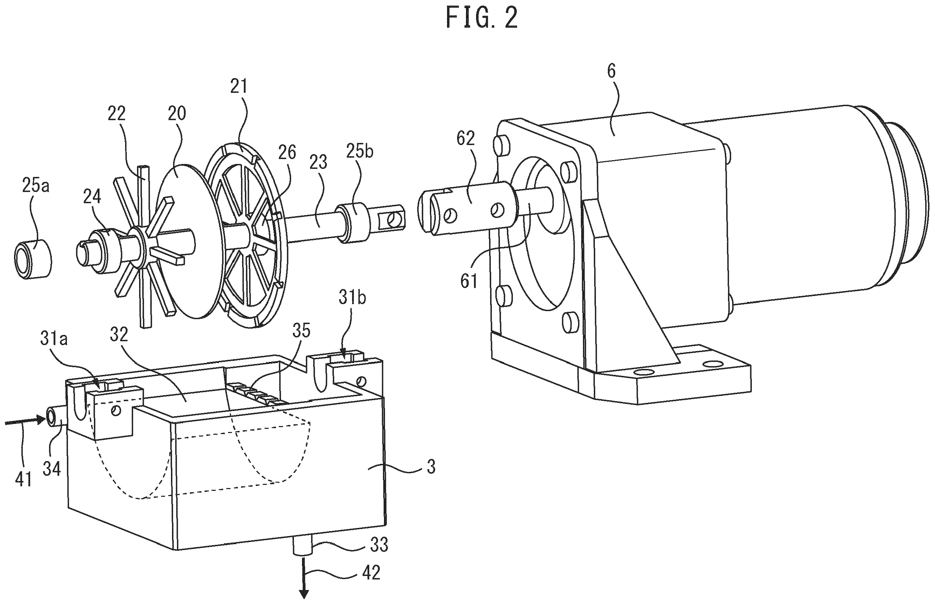

FIG. 2 is a perspective view illustrating an exemplary configuration of the cell culture apparatus of the invention, in which some members contained in the exemplary configuration in FIG. 1 are depicted independently from each other. In this embodiment, the cell culture section 2 illustrated in FIG. 1 is composed of a porous polymer film 20, a porous polymer film support 21 and a porous polymer film clamp 22, with the porous polymer film being supported and sandwiched between the support and the clamp, and a shaft 23 penetrating each of them. A shaft 23 is provided with a flange 26 which prevents the porous polymer film support 21 from being displaced toward the rotary motor 6. The porous polymer film support 21 may be directly formed on the shaft 23. A clamp ring 24 may be provided outside of the porous polymer film 22 clamp. The clamp ring 24 may apply pressure toward the porous polymer film support 21, thereby fixing the porous polymer film. In another embodiment, it may be configured that the clamp ring 24 has a female screw and at least a part of the shaft 23 has a male screw, so as to be pressed by screwing. In another embodiment, there is provided a screw hole in a part of the clamp ring 24, a male screw may be inserted in the screw hole to be screwed vertically to the shaft 23.

The porous polymer film may be used singularly or two or more sheets of them may be stacked for use. Using two or more sheets of them may increase a space in which cell culture may be conducted. Thus, more cells may be grown.

In an embodiment of the present invention, bearing bushes (25a, 25b) are provided on the shafts 23 on both sides of the cell culture section 2. The bearing bushes (25a, 25b) each are fit into bearings (31a, 31b) provided in a medium tank 3. The bearings (31a, 31b) may stably rotate the shaft 23 without displacing the shaft.

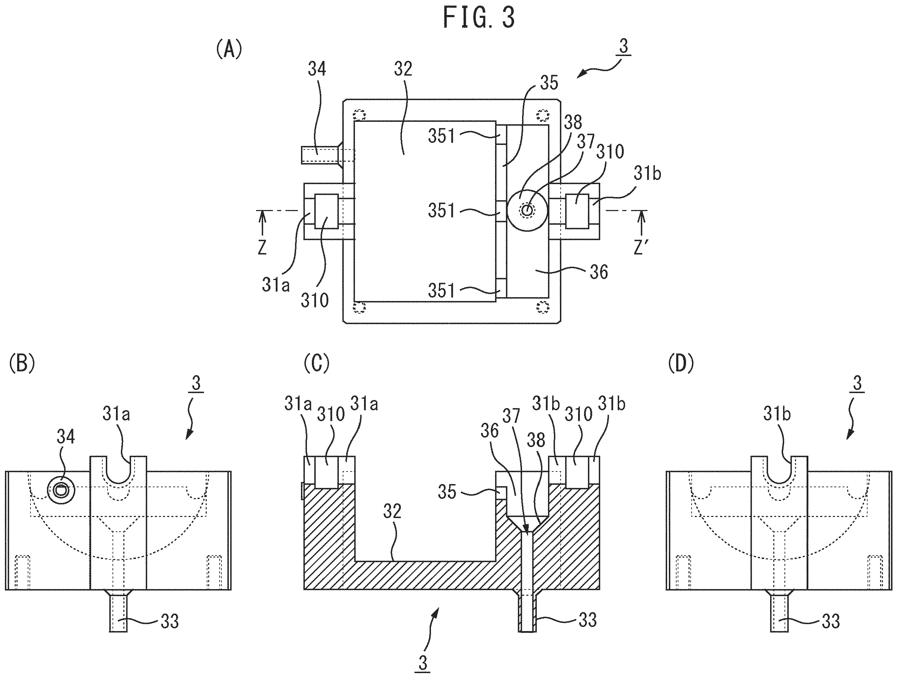

FIG. 3 is a diagram illustrating a culture tank 3 which constitutes a cell culture apparatus 1 of the invention. FIG. 3(C) is a cross sectional view taken along Z-Z' axis in FIG. 3(A), FIGs. 3(B) and (D) are a left side view and a right side view of FIG. 3(A), respectively. The above bearings (31a, 31b) have bearing bushes (25a, 25b) being fit therein, and are provided with a bearing depression portion 310 with a depth so as not to interfere the rotation of the bearing bushes (25a, 25b). Therefore, the shaft 23 is prevented from being detached from the culture tank 3. A part of the medium storage unit 32 is provided with a medium storage inner wall 35, and the medium storage inner wall 35 is provided with medium storage inner wall depressions 351 at a plurality of points. The size, shape or the like of the medium storage inner wall 35 and the medium storage inner wall depression 351 may determine the amount of medium contained in the medium storage unit. When the cell culture section 2 is placed, a medium corresponding to the volume of the cell culture section 2 will overflow into the medium overflow section. The overflow of the medium passes through a medium discharging port 37 provided in a medium overflow section 36 and is discharged out of a medium tank 3 through a medium discharging pipe 33. The circumference of the medium discharging port 37 forms a taper 38 and is processed for easy discharge of the medium. The form of the bottom of the medium storage unit 32 may be any form which does not interfere the medium flow when the cell culture section 2 is applied and rotated. In the present embodiment, since the cell culture section 2 has a disk-like shape, its side face has a semicircular column-like shape having a radius larger than that of the cell culture section 2.

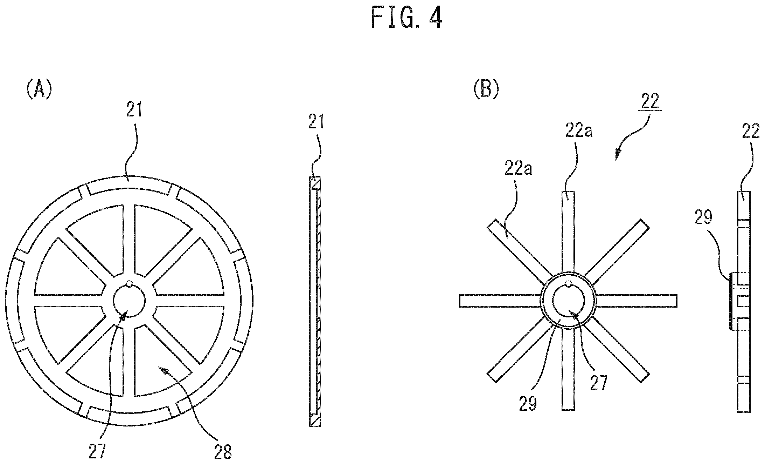

FIG. 4 is a diagram illustrating an embodiment of a cell culture section 2 which is used in the cell culture apparatus 1 of the present invention. (A) is a planar and cross sectional views of a porous polymer film support 21, (B) is a planar and cross sectional views of a porous polymer film clamp 22. The porous polymer film support 21 is provided with a shaft penetrating port 27 for a shaft 23 to penetrate therethrough. The diameter of the shaft penetrating port 27 is determined depending on the diameter of the shaft 23 and is not particularly limited. The porous polymer film support 21 is provided with a medium passing port 28 which is to be fixed so as to supply a medium and air (oxygen) to a porous polymer film to be fixed. Any shape and area of the medium passing port 28 may be used without limiting to the shape and diameter illustrated in the drawing, so long as it is enough to sufficiently supply a medium and air (oxygen) to the porous polymer film.

In this embodiment, the porous polymer film clamp 22 includes a plurality of spokes 22a radially extending from a porous polymer film clamp ring 29 having a shaft penetrating port 27. The length of the spoke 22a is equal to or shorter than the radius of the porous polymer film support 21. The porous polymer film clamp 22 may be combined with a porous polymer film support 21 to make it possible to fix a porous polymer film 20. The porous polymer film clamp 22 may be any one which is combined with a porous polymer film support 21 to make it possible to fix the porous polymer film, and is not limited to the shape illustrated in the drawings. For example, two or more sheets of porous polymer films 20 may be stacked and sandwiched between the porous polymer film support 21 and the porous polymer film clamp 22. A cell culture section 2 is a set of a porous polymer film 20, a porous polymer film support 21, and a porous polymer film clamp 22. Culture may be conducted by connecting two or more of the cell culture sections 2. FIG. 1 illustrates a cell culture apparatus 1 composed of five sets of the cell culture sections 2 connecting each other.

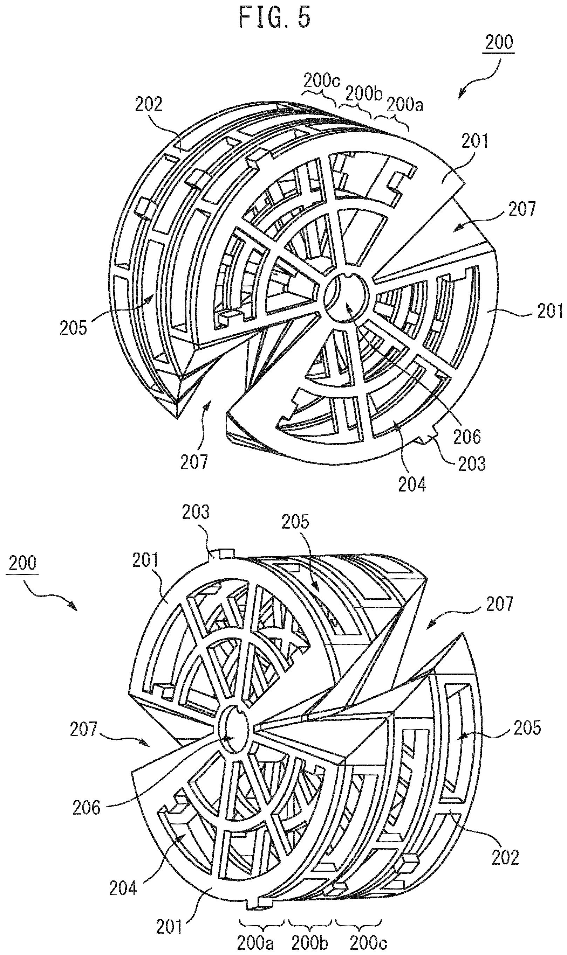

FIG. 5 is a diagram illustrating another embodiment of a cell culture section 2 usable for the cell culture apparatus 1 of the present invention. Both of the upper and lower panels are diagrams illustrating the same cell culture section 2 as viewed from the different angles. The cell culture section 2 of this embodiment is cylindrical containers (200, 200a, 200b, 200c). An end face 201 of the cylindrical containers (200, 200a, 200b, 200c) includes one or more medium flow inlets 204, and a side face 202 of the cylindrical containers (200, 200a, 200b, 200c) includes one or more medium flow inlets 205. Although not illustrated in this drawing, a porous polymer film is contained in the cylindrical containers (200, 200a, 200b, 200c). Since the cell culture section 2 has a shape of the cylindrical containers (200, 200a, 200b, 200c), it may contain arbitrary amount of porous polymer film. The shape and area of the medium flow inlets 204 and 205 are not limited by the shape illustrated in this drawing. The inlets having shape and area sufficient, to supply a medium and air (oxygen) to a porous polymer film and to prevent the porous polymer film contained therein from being detached, may be employed.

An end face 201 of the cylindrical containers (200, 200a, 200b, 200c) has a function as a lid of the cylindrical containers (200, 200a, 200b, 200c), and may be opened by means of hook 203 provided on the end face 201. Accordingly, a porous polymer film may be contained in cylindrical containers (200, 200a, 200b, 200c). The height of the side face 202 is not particularly limited but is of any height so as to contain a desired amount of a porous polymer film. FIG. 5 illustrates three cylindrical containers (200, 200a, 200b, 200c) connected each other. A desired number of cylindrical containers (200, 200a, 200b, 200c) may be connected. Accordingly, a desired number of cells may be cultured.

The cylindrical containers (200, 200a, 200b, 200c) of this embodiment have a shaft penetrating port 206, center of which a shaft 23 penetrates. The diameter of the shaft penetrating port 206 is determined depending on the diameter of the shaft 23 and is not particularly limited.

The cylindrical containers (200, 200a, 200b, 200c) of the present embodiment include one or more helical flow paths 207 which communicate a part of the one end face of the cylindrical containers (200, 200a, 200b, 200c) and the other end face of the cylindrical containers (200, 200a, 200b, 200c). In this embodiment, the connected cylindrical container 200 has a continuous helical flow path 207 is formed in the direction toward the shaft penetrating port 206. The helical flow path 207 is also formed on the respective cylindrical container (200a, 200b, 200c) unit. When two or more helical flow paths 207 are provided, all the helical flow path 207 may be formed so as to be parallel. Since the helical flow path 207 is a space which does not form cylindrical containers (200, 200a, 200b, 200c), the space cannot contain a porous polymer film. When the cylindrical containers (200, 200a, 200b, 200c) including the helical flow path 207 are rotated in a medium in a culture tank 3, a medium is transferred from one direction to the other direction through the helical flow path 207. In this way, the medium in the culture tank 3 is stirred. Accordingly, the concentration of the various kinds of components in the medium in the culture tank 3 may become constant.

When a helical flow path 207 is provided in the cylindrical containers (200, 200a, 200b, 200c), the cylindrical containers (200, 200a, 200b, 200c) might not be fixed with a shaft 23. In this case, the cylindrical containers (200, 200a, 200b, 200c) themselves rotate around the shaft 23. A medium flow generated from one direction to the other direction in the medium tank 3 causes the medium to flow into the helical flow path 207, thereby making it possible to generate rotational movement of the cylindrical containers (200, 200a, 200b, 200c). By appropriately adjusting the number, the angle, the shape or the like of the helical flow path 207, the rotation at arbitrary speed may become possible.

Examples of the cell culture section 2 and cylindrical containers (200, 200a, 200b, 200c) used in an embodiment of the present invention include, for example, polystyrene, polycarbonate, polymethyl methacrylate, polyethylene terephthalate; metals such as stainless steel (also referred to as "stainless"), but not limited thereto so long as not to affect cell culture.

In another embodiment of the present invention, the helical flow path 207 is not provided in cylindrical containers (200, 200a, 200b, 200c). In this case, the cylindrical containers (200, 200a, 200b, 200c) are fixed to a shaft 23 penetrating through a shaft penetrating port 206.

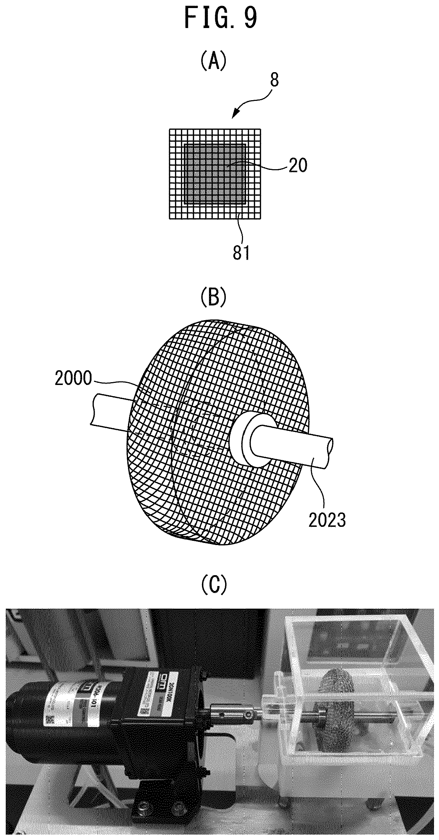

A cell culture section 2 used in an another embodiment of the present invention may be a stainless steel mesh cylindrical container 2000 fabricated with a stainless steel mesh as illustrated in FIG. 9 (B). Having a stainless mesh-like shape makes it possible to cause a medium to flow into/out of a stainless mesh cylindrical container 2000. In addition, stainless steel mesh enables sterilization using a sterilization means such as dry heat sterilization while retaining porous polymer film inside.

Although not illustrated, in another embodiment of the invention, a gas phase zone of a cell culture section 2 may be provided with a medium droplet supply means which supplies medium in the form of fine droplets. A medium in the form of fine droplets may be supplied from a medium droplet supply means, thereby supplying it to a porous polymer film in a cell culture section 2. Accordingly, a porous polymer film on which cells grow may be supplied while being exposed to a gas phase. In the present invention, a medium droplet supply means may be any one by which a medium may be supplied in the form of droplet, and is not limited by the size of the droplet to be supplied. In this specification, a medium droplet supply means does not limit the size of the droplet to be supplied and thus encompass in meaning an apparatus which supplies a medium including a shower-like medium and a mist-like medium. Preferably, a medium droplet supply means may be arranged so as to enable spraying a cell culture section 2 to a gas phase-exposed zone.

In this specification, a "dropletized medium" means a mist-like or dropletized medium, and a medium which is ready for jetting or spraying onto a porous polymer film used for the present invention. The diameter of the dropletized medium is not limited but may be a fine mist-like dropletized medium so as to be floatable in an air without being freely fallen by the gravity. The diameter of the mist-like dropletized medium may be about 1 $\mu$m to 100 $\mu$m, or may be even smaller. In addition, a dropletized medium may be, for example, droplet-like medium which freely falls by the gravity, for example more than 100 $\mu$m. Examples of a method for forming droplet of a medium include, for example, a method for forming droplet by a known means, for example, a medium may be dropletized using, for example, a mist-like nozzle or a shower-like nozzle. However, a method for dropletizing is preferably a method which does not alter components of the medium. For example, a method in which the medium is evaporated is excluded from the droplet forming methods.

In an embodiment of the present invention, a dropletized medium is applied to a porous polymer film with a cell supported thereon. A dropletized medium is passed through a gas phase before it reaches to a porous polymer film. Thus, oxygen is dissolved in a medium. In this way, a medium having a sufficient amount of oxygen is continuously supplied, and it is possible to perform culture without cells becoming ischemic. In addition, since a porous polymer film is always exposed to a gas phase, it is possible for a medium which adheres to the porous polymer film to always intake oxygen, enabling culture while efficiently supplying oxygen.

The porous polymer film used in the present embodiment may be applied to the cell culture section 2 with:

i) being folded up;
ii) being wound into a roll-like shape;
iii) sheets or pieces thereof being concatenated with a thread-like structure; and/or
iv) being tied together into a rope-like shape. The porous polymer film used in an embodiment of the present invention may be applied to the cell culture section 2 with: v) two or more of them being stacked. By forming the porous polymer films into shapes such as i) to v), it is possible to place a large amount of porous polymer films into a fixed volume of cell culture medium.

As the porous polymer film used in the present embodiment, a modularlized porous polymer film (hereinafter referred to as a "modularlized porous polymer film") may be used. In this specification, "a modularlized porous polymer film" means a porous polymer film contained in a casing. It should be noted that the phrase "a modularlized porous polymer film" may be expressed simply as "a module", both expression can be used interchangeably to indicate the same meaning.

A casing which is contained in a modularlized porous polymer film used in the embodiment of the present invention has two or more cell culture medium flow inlets and such a cell culture medium flow inlet makes the medium to flow in or out of the casing. The diameter of the cell culture medium flow inlet of the casing is preferably larger than the diameter of the cell so as to enable cell to flow into the casing. In addition, the diameter of the cell culture medium flow inlet is preferably smaller than the diameter of the porous polymer film which flows out through the cell culture medium flow inlet. The diameter smaller than the diameter of the porous polymer film which flows out may be appropriately selected depending on the shape and size of the porous polymer film contained in the casing. For example, in the case where the porous polymer film has string-like shape, the diameter is not particularly limited so long as it is smaller than the width of the shorter side of the porous polymer film so that the porous polymer film is prevented from flowing out. It is preferred to provide as many cell culture medium flow inlets as possible so that the cell culture medium may be easily supplied into and/or discharged out of the casing. It is preferably 5 or more, preferably 10 or more, preferably 20 or more, preferably 50 or more, and preferably 100 or more. As the cell culture medium flow inlet, the casing may have a mesh-like structure in part or in whole. Moreover, the casing itself may be mesh-like. In the present invention, examples of mesh-like structure include, but not limited to, lattices including longitudinal, transverse, and/or oblique elements wherein individual apertures form cell culture medium flow inlet which allows the fluid to pass therethrough.

Examples of a casing of a modularized porous polymer film used in an embodiment of the present invention include, for example, polystyrene, polycarbonate, polymethyl methacrylate, polyethylene terephthalate; metals such as stainless steel, but not limited thereto, and are not particularly limited so long as they have no effect on cell culture.

A modularlized porous polymer film used in an embodiment of the present invention is contained in the casing with;

(i) the two or more independent porous polymer films being aggregated;
(ii) the porous polymer films being folded up;
(iii) the porous polymer films being wound into a roll-like shape; and/or
(iv) the porous polymer film being tied together into a rope-like shape;

wherein it is possible to apply the modularlized porous polymer film to the cell culture section 2.

In this specification, "two or more independent porous polymer films are aggregated and contained within a casing" means that two or more independent porous polymer films are aggregated and contained in a predetermined space surrounded by a casing. According to the present invention, the two or more independent porous polymer films may be immovably fixed by fixing at least one point of the porous polymer film to at least one point of the casing by an arbitrary method. The porous polymer film may be kept immovable in the casing. In addition, the two or more independent porous polymer films may be fragments. A fragment may take an arbitrary shape such as circular, elliptical, quadrilateral, triangular, polygonal or string-like shape, and is preferably a substantially square shape. In the present invention, the fragments may be of any size. When it has a substantially square shape, the side may be any length, but, for example, preferably 80 mm or less, preferably 50 mm or less, more preferably 30 mm or less, still more preferably 20 mm or less, and may be 10 mm or less. In addition, when the fragments of the porous polymer film are substantially square, it may be formed so that length of each side may match the inner wall or may be shorter than each side of the inner wall (e.g. shorter by about 0.1 mm to 1 mm), rendering the porous polymer film immovable in the casing. This can protect cells to be grown in the porous polymer film from stress to be applied.

In this specification, "the porous polymer films being folded up" means a porous polymer film which is folded up in the casing, and thus it is rendered immovable in the casing by frictional force between each surfaces of the porous polymer film and/or the inner surface of the casing. In this specification, "being folded up" may indicate the pours polymer film being creased or creaseless.

In this specification, "the porous polymer films being wound into a roll-like shape" means the porous polymer film being wound into a roll-like shape and thus it is rendered immovable in the casing by frictional force between each surfaces of the porous polymer film and/or the inner surface of the casing. Moreover, in the present invention, the porous polymer film being twisted together into a rope-like shape means, for example, more than one porous polymer films in rectangle strip shape are knitted into a rope-shape by arbitrary method, rendering the porous polymer films immovable by the mutual frictional force of the porous polymer films. It is also possible that (i) the two or more independent porous polymer films being aggregated; (ii) the porous polymer films being folded up; (iii) the porous polymer films being wound into a roll-like shape; and/or (iv) the porous polymer film being tied together into a rope-like shape may be combined and contained within a casing.

In this specification, "the porous polymer film being immovable in the casing" means that the porous polymer film is contained in the casing so that the porous polymer film is continued to be morphologically unchanged during culturing the cell culture module in the cell culture medium. In other words, the porous polymer film itself is prevented from continual waving movement by fluid. Since the porous polymer film is kept immovable in the casing, the cell being grown in the porous polymer film is protected from stress to be applied, enabling stable cell culture without cells being killed by apoptosis.

3. Cell culture method using cell culture apparatus

<Step for applying cells to porous polymer film>

There are no particular restrictions on the specific steps for application of the cells usable for the present invention to the porous polymer film. It is possible to carry out the steps described throughout the present specification, or to employ any desired method suited for applying cells to a film-like support. Application of cells to the porous polymer film in the method of the invention includes, but is not limited to, the following modes.

(A) A mode including a step of seeding cells on the surface of a porous polymer film;
(B) A mode including steps of
placing a cell suspension on the dried surface of a porous polymer film, allowing it to stand, or moving the porous polymer film to promote efflux of the liquid, or stimulating part of the surface to cause absorption of the cell suspension into the film, and
retaining the cells in the cell suspension inside the film and allowing the water to flow out;
and
(C) A mode including steps of:

wetting one or both sides of a porous polymer film with a cell culture medium or a sterilized liquid,
loading a cell suspension into the wetted porous polymer film, and
retaining the cells in the cell suspension inside the film and allowing the water to flow out.

Mode (A) includes a step of directly seeding cells or a cell mass on the surface of a porous polymer film. Alternatively, it includes a mode of placing a porous polymer film in a cell suspension and wetting the porous polymer film from the surface thereof with the cell culture medium.

Cells seeded on the surface of a porous polymer film adhere to the porous polymer film and infiltrate into the interiors of the pores. Preferably, the cells adhere to the porous polymer film without applying any particular exterior physical or chemical force. The cells that have been seeded on the surface of the porous polymer film can stably grow and proliferate on the surface and/or in the interior of the film. The cells may be in a variety of different forms, depending on the location of the film used for growth and proliferation.

For mode (B), a cell suspension is placed on the dried surface of a porous polymer film. The porous polymer film is allowed to stand, or the porous polymer film is moved to promote efflux of the liquid, or part of the surface is stimulated to cause absorption of the cell suspension into the film, so that the cell suspension permeates into the film. While it is not our intention to be constrained by theory, this is believed to be due to the properties of each of the surface forms of the porous polymer film. According to this mode, the cells are absorbed and seeded in the locations of the film where the cell suspension has been loaded.

Alternatively, as according to mode (C), after all or a portion of one or both sides of the porous polymer film has been wetted with the cell culture medium or sterilized liquid, the cell suspension may be loaded into the wetted porous polymer film. This will significantly increase the transit rate of the cell suspension.

For example, a method of wetting a portion of the film edges, for the main purpose of preventing fly loss of the film, may be used (hereunder referred to as "single-point wetting method"). The single-point wetting method is nearly the same as the dry method (mode (B)) in which the film essentially is not wetted. However, it is possible that cell solution permeation through the film is more rapid at the small wetted portions. There may also be used a method in which all of one or both sides of the porous polymer film that have been thoroughly wetted (hereunder this will also be referred to as "wet film") is loaded with a cell suspension (this will hereunder be referred to as "wet film method"). In this case, the entire porous polymer film has a greatly increased transit rate for the cell suspension.

According to modes (B) and (C), the cells in the cell suspension are retained in the film, while the water flows out. This allows treatment such as increasing the concentration of cells in the cell suspension and flowing out of unwanted non-cellular components together with the water.

Mode (A) will also be referred to as "natural seeding", and modes (B) and (C) as "suction seeding".

Preferably, but not restrictively, the viable cells are selectively retained in the porous polymer film. Thus, according to a method of a preferred embodiment of the invention, the viable cells are retained in the porous polymer film, and the dead cells preferentially flow out together with the water.

The sterilized liquid used for mode (C) is not particularly restricted, and may be a sterilized buffering solution or sterilized water. A buffering solution may be, for example, (+) or (-) Dulbecco's PBS, or (+) or (-) Hank's Balanced Salt Solution. Examples of buffering solutions are listed in Table 1 below.

[Table 1]

| Component | Concentration (mmol/L) | Concentration (g/L) |
|---|---|---|
| NaCl | 137 | 8,00 |
| KCl | 2.7 | 0.20 |
| $Na_2HPO_4$ | 10 | 1.44 |
| $KH_2PO_4$ | 1.76 | 0.24 |
| pH(-) | 7.4 | 7.4 |

In the method of the invention, application of cells to the porous polymer film further includes a mode of adding adherent cells in a floating state as a suspension together with the porous polymer film, to adhere the cells with the film (entangling) may be included. For example, for application of the cells to the porous polymer film in the method of the invention, the cell culture medium, the cells and one or more of the porous polymer films may be placed in the cell culturing vessel. When the cell culture medium is a liquid, the porous polymer film is in a floating state in the cell culture medium. The cells can adhere to the porous polymer film due to the properties of the porous polymer film. Thus, even with cells that are not suited for natural suspension culture, the porous polymer film allows culturing in a floating state in the cell culture medium. The cells preferably adhere to the porous polymer film. Here, "adhere spontaneously" means that the cells are retained on the surface or in the interior of the porous polymer film without applying any particular exterior physical or chemical force.

Application of cells to the porous polymer film described above may be used in combination of two or more methods. For example, a cell may be applied to a porous polymer film by combining two or more methods of the modes (A) to (C). It is possible to apply and culture a porous polymer film supporting cells to a cell culture section 2 in the aforementioned cell culture apparatus 1.

In addition, a medium containing a suspended cell may be previously added dropwise and seeded to a cell culture section in which a porous polymer film is contained from a cell supply means 3.

In this specification, a "suspended cell" encompasses cells obtained by forcing to suspend an adherent cell in a medium with a proteolytic enzyme such as trypsin, and cells which can be suspension-cultured in a medium, obtained by the known conditioning step, and etc..

The types of the cells which may be used for the present invention may be selected from the group consisting of animal cells, insect cells, plant cells, yeast cells and bacteria. Animal cells are largely divided into cells from animals belonging to the subphylum Vertebrata, and cells from non-vertebrates (animals other than animals belonging to the subphylum Vertebrata). There are no particular restrictions on the source of the animal cells, for the purpose of the present specification. Preferably, they are cells from an animal belonging to the subphylum Vertebrata. The subphylum Vertebrata includes the superclass Agnatha and the superclass Gnathostomata, the superclass Gnathostomata including the class Mammalia, the class Aves, the class Amphibia and the class Reptilia. Preferably, they are cells from an animal belonging to the class Mammalia, generally known as mammals. Mammals are not particularly restricted but include, preferably, mice, rats, humans, monkeys, pigs, dogs, sheep and goats.

The types of animal cells or plant cells that may be used for the invention are not particularly restricted, but are preferably selected from the group consisting of pluripotent stem cells, tissue stem cells, somatic cells and germ cells.

The term "pluripotent stem cells", in this specification, is intended as a comprehensive term for stem cells having the ability to differentiate into cells of any tissues (pluripotent differentiating power). While not restrictive, pluripotent stem cells include embryonic stem cells (ES cells), induced pluripotent stem cells (iPS cells), embryonic germ cells (EG cells) and germ stem cells (GS cells). They are preferably ES cells or iPS cells. Particularly preferred are iPS cells, which are free of ethical problems, for example. The pluripotent stem cells used may be any publicly known ones, and for example, the pluripotent stem cells described in WO2009/123349 (PCT/JP2009/057041) may be used.

The term "tissue stem cells" refers to stem cells that are cell lines capable of differentiation but only to limited specific tissues, though having the ability to differentiate into a variety of cell types (pluripotent differentiating power). For example, hematopoietic stem cells in the bone marrow are the source of blood cells, while neural stem cells differentiate into neurons. Additional types include hepatic stem cells from which the liver is formed and skin stem cells that form skin tissue. Preferably, the tissue stem cells are selected from among mesenchymal stem cells, hepatic stem cells, pancreatic stem cells, neural stem cells, skin stem cells and hematopoietic stem cells.

The term "somatic cells" refers to cells other than germ cells, among the cells composing a multicellular organism. In sexual reproduction, these are not passed on to the next generation. Preferably, the somatic cells are selected from among hepatocytes, pancreatic cells, muscle cells, bone cells, osteoblasts, osteoclasts, chondrocytes, adipocytes, skin cells, fibroblasts, pancreatic cells, renal cells and lung cells, or blood cells such as lymphocytes, erythrocytes, leukocytes, monocytes, macrophages or megakaryocytes.

The term "germ cells" refers to cells having the role of passing on genetic information to the succeeding generation in reproduction. These include, for example, gametes for sexual reproduction, i.e. the ova, egg cells, sperm, sperm cells, and spores for asexual reproduction.

The cells may also be selected from the group consisting of sarcoma cells, established cell lines and transformants. The term "sarcoma" refers to cancer occurring in non-epithelial cell-derived connective tissue cells, such as the bone, cartilage, fat, muscle or blood, and includes soft tissue sarcomas, malignant bone tumors and the like. Sarcoma cells are cells derived from sarcoma. The term "established cell line" refers to cultured cells that are maintained in vitro for long periods and reach a stabilized character and can be semi-permanently subcultured. Cell lines derived from various tissues of various species including humans exist, such as PC12 cells (from rat adrenal medulla), CHO cells (from Chinese hamster ovary), HEK293 cells (from human embryonic kidney), HL-60 cells (from human leukocytes) and HeLa cells (from human cervical cancer), Vero cells (from African green monkey kidney epithelial cells), MDCK cells (from canine renal tubular epithelial cells), HepG2 cells (from human hepatic cancer), BHK cells (newborn hamster kidney cell), NIH3T3 cells (from mouse fetal fibroblast cells). The term "transformants" refers to cells with an altered genetic nature by extracellularly introduced nucleic acid (DNA and the like).

In this specification, an "adherent cell" is generally a cell which is required to adhere itself on an appropriate surface for growth, and is also referred to as an attachment cell or an anchorage-dependent cell. In certain embodiments of the present invention, the cells used are adherent cells. The cells used for the present invention are adherent cells, more preferably cells which may be cultured even as a suspension in a medium. The adherent cells which can be suspension cultured may be obtained by conditioning the adherent cells to a state suitable for suspension culture, and include, for example, CHO cells, HEK293 cells, Vero cells, NIH3T3 cells, and cell lines derived from these cells.

FIG. 1 represents a model diagram of cell culturing using a porous polymer film. FIG. 1 serves merely for illustration and the elements are not drawn to their actual dimensions. In the cell culture method of the invention, application of cells and culturing are carried out on a porous polymer film, thereby allowing culturing of large volumes of cells to be accomplished since large numbers of cells grow on the multisided connected pore sections on the inside, and the surfaces on the porous polymer film. Moreover, in the cell culture method of the invention, it is possible to culture large volumes of cells while drastically reducing the amount of medium used for cell culturing compared to the prior art. For example, large volumes of cells can be cultured even when all or a portion of the porous polymer film is not in contact with the liquid phase of the cell culture medium. In addition, the total volume of the cell culture medium in the cell culture vessel, with respect to the total porous polymer film volume including the cell survival zone, can be significantly reduced.

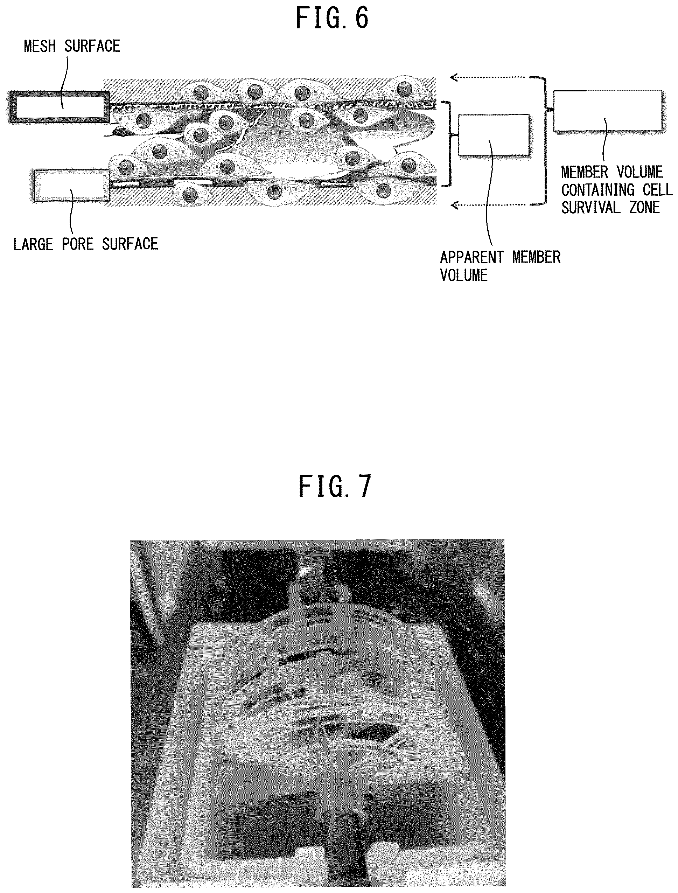

Throughout the present specification, the volume of the porous polymer film without cells, that occupies the space including the volume between the interior gaps, will be referred to as the "apparent porous polymer film volume" (see, FIG. 6). In the state where the cells are applied to the porous polymer film and the cells have been carried on the surface and the interior of the porous polymer film, the total volume of the porous polymer film, the cells and the medium that has wetted the porous polymer film interior, which is occupying the space therein, will be referred to as the "porous polymer film volume including the cell survival zone" (see, FIG. 6). When the porous polymer film has a film thickness of 25 $\mu$m, the porous polymer film volume including the cell survival zone is a value of at maximum about 50% larger than the apparent porous polymer film volume. In the method of the invention, a plurality of porous polymer films may be housed in a single cell culture vessel for culturing, in which case the total sum of the porous polymer film volume including the cell survival zone for each of the plurality of porous polymer films supporting the cells may be referred to simply as the "total sum of the porous polymer film volume including the cell survival zone".

Using the method of the invention, cells can be satisfactorily cultured for a long period of time even under conditions in which the total volume of the cell culture medium in the cell culture vessel is 10,000 times or less of the total sum of the porous polymer film volume including the cell survival zone. Moreover, cells can be satisfactorily cultured for a long period of time even under conditions in which the total volume of the cell culture medium in the cell culture vessel is 1,000 times or less of the total sum of the porous polymer film volume including the cell survival zone. In addition, cells can be satisfactorily cultured for a long period of time even under conditions in which the total volume of the cell culture medium in the cell culture vessel is 100 times or less of the total sum of the porous polymer film volume including the cell survival zone. In addition, cells can be satisfactorily cultured for a long period of time even under conditions in which the total volume of the cell culture medium in the cell culture vessel is 10 times or less of the total sum of the porous polymer film volume including the cell survival zone.

In other words, according to the invention, the space (vessel) used for cell culturing can be reduced to an absolute minimum, compared to a conventional cell culture apparatus for performing two-dimensional culture. Furthermore, when it is desired to increase the number of cells cultured, the cell culturing volume can be flexibly increased by a convenient procedure including increasing the number of layered porous polymer films. In a cell culture apparatus comprising a porous polymer film to be used for the invention, the space (vessel) in which cells are cultured and the space (vessel) in which the cell culture medium is stored can be separate, and the necessary amount of cell culture medium can be prepared according to the number of cells to be cultured. The space (vessel) in which the cell culture medium is stored can be increased or decreased according to the purpose, or it may be a replaceable vessel, with no particular restrictions.

In the cell culture method of the invention, culturing in which the number of cells in the cell culture vessel after culturing using the porous polymer film reaches $1.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more $5.0 \times 10^7$ or more, $1.0 \times 10^8$ or more, $2.0 \times 10^8$ or more, $5.0 \times 10^8$ or more, $1.0 \times 10^9$ or more, $2.0 \times 10^9$ or more, or $5.0 \times 10^9$ or more per milliliter of medium, assuming that all of the cells are evenly dispersed in the cell culture medium in the cell culture vessel, is mentioned.

It should be noted that as a method for measuring cell count during or after culture, various known methods may be used. For example, as the method for counting the number of cells in the cell culture vessel after culturing using the porous polymer film, assuming that the cells are evenly dispersed in the cell culture medium in the cell culture vessel, any publicly known method may be used. For example, a cell count method using CCK8 may be suitably used. Specifically, a Cell Counting Kit 8 (a solution reagent, commercially available from Dojindo Laboratories) (hereunder referred to as "CCK8") may be used to count the number of cells in ordinary culturing without using a porous polymer film, and the correlation coefficient between the absorbance and the actual cell count is determined. Subsequently, the cells are applied, the cultured porous polymer film may be transferred to CCK8-containing medium and stored in an incubator for 1 to 3 hours, and then the supernatant is extracted and its absorbance is measured at a wavelength of 480 nm, and the cell count is determined from the previously calculated correlation coefficient.

In addition, from another point of view, for example, "mass culturing of cells" may refer to culturing in which the number of cells in the cell culture vessel after culturing using the porous polymer film reaches $1.0 \times 10^5$ or more, $2.0 \times 10^5$ or more, $1.0 \times 10^6$ or more, $2.0 \times 10^6$ or more, $5.0 \times 10^6$ or more, $1.0 \times 10^7$ or more, $2.0 \times 10^7$ or more or $5.0 \times 10^7$ or more, $1.0 \times 10^8$ or more, $2.0 \times 10^8$ or more, or $5.0 \times 10^8$ or more, per square centimeter of porous polymer film. The number of cells contained per square centimeter of porous polymer film may be appropriately measured using a publicly known method, such as with a cell counter.

EXAMPLES

Hereinafter, the present invention will be described in more detail based on Examples. The present invention is not limited to the following Examples. Those skilled in the art can easily modify/modify the present invention based on the description of the present specification, and they are included in the technical scope of the present invention.

The porous polyimide films used in the following Examples were prepared by forming a polyamic acid solution composition including a polyamic acid solution obtained from 3,3',4,4'-biphenyltetracarboxylic dianhydride (s-BPDA) as a tetracarboxylic acid component and 4,4'-diaminodiphenyl ether (ODA) as a diamine component, and polyacrylamide as a coloring precursor, and performing heat treatment at 250°C or higher. The resulting porous polyimide film was a three-layer structure porous polyimide film having a surface layer A and a surface layer B, the surface layers having a plurality of pores, and a macrovoid layer sandwiched between the surface layers A and B; wherein the average pore diameter of the pore present on the surface layer A was 6 $\mu$m, the average pore diameter of the pore present on the surface layer B was 46 $\mu$m, and the film thickness was 25 $\mu$m, and the porosity was 73%.

[Example 1]

Gas phase-exposed type rotation culture apparatus

Conditioned/suspended anti-human IL-8 antibody producing CHO-DP12 cells (ATCC CRL-12445) were suspension-cultured using a medium (BalanCD (Trademark) CHO GROWTH A) and culture was continued until vial cell count per mL was $1.3 \times 106$. In a gas phase-exposed type rotation culture apparatus (vertical drum type, without spiral flow channel) illustrated in FIG. 7, 18 modules were placed, the module having a mantle (casing) formed with a nylon mesh (30 #, mesh opening 547 $\mu$m) and having a fixed amount (20 $cm^2$ per module) of porous polyimide film aseptically added and sealed therein, and prepared ready-for-rotation. After 40 mL of the suspension culture medium was added to the upper sump (corresponding to a medium tank 3 in FIG. 1), and the suspension culture medium was wetted to at a slow speed as low as 6 rpm. After leaving the entire apparatus including this rotating part in a $CO_2$ incubator for 5 hours, suspension culture medium in the upper sump (corresponding to a medium discharging tank 3 in FIG. 1) was removed and 500 mL of medium (IMDM containing 2% FBS) was added from the lower sump in which the medium was pooled, while continuing the rotation of the module, and the medium was circulated via a tube pump at a rate of 10 ml/min. When culture was performed for 7 days, cells with a total cell count of $3.2 \times 10^5$ at a cell density of $1.0 \times 10^8$ cells/$cm^2$ were observed.

[Example 2]

Gas phase-exposed type rotation culture apparatus

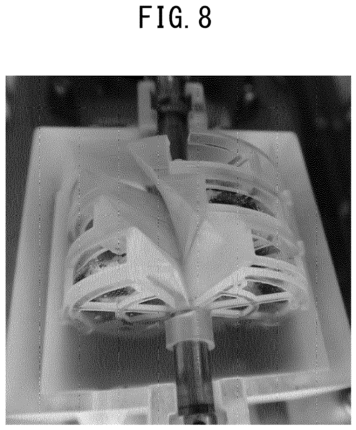

Conditioned/suspended anti-human IL-8 antibody producing CHO-DP12 cells (ATCC CRL-12445) were suspension-cultured using a medium (BalanCD (Trademark) CHO GROWTH A) and culture was continued until vial cell count per mL was $1.3 \times 10^6$. In a gas phase rotation culture apparatus (notched drum type, with spiral flow channel) illustrated in FIGs. 5 and 8, 18 modules were placed, the module having a mantle (casing) formed with a nylon mesh (30 #, mesh opening 547 $\mu$m) and having a fixed amount (20 $cm^2$ per module) of porous polyimide film aseptically added and sealed therein, and prepared ready-for-rotation. After 40 mL of the suspension culture medium was added to the upper sump, and the rotating part was wetted with the suspension culture medium at a slow speed as low as 6 rpm. After leaving the entire apparatus including this rotating part in a $CO_2$ incubator overnight, suspension culture medium in the upper sump (corresponding to a culture tank 3 in FIG. 1) was removed and 500 mL of medium (IMDM containing 2% FBS) was added from the lower sump (corresponding to a medium discharging tank 4 in FIG. 1) in which the medium was pooled, while continuing the rotation of the module, and the medium was circulated via a tube pump at a rate of 10 ml / min. When culture was performed for 7 days, cells with a total cell count of $2.4 \times 10^5$ at a cell density of $8.5 \times 10^7$ cells/$cm^2$ were observed.

[Example 3]

<Fabrication of modularized porous polymer film having a stainless steel casing (hereinafter referred to as "metal module") and a stainless steel cell culture section (hereinafter referred to as "metal drum")>

In order to fully utilize the heat resistance of the porous polyimide film and complete the sterilization operation by a simple bulk dry heat sterilization, a metal module composed of a stainless steel mesh casing, a liner, and a porous polyimide film was prepared (see, FIG. 9 (A)). Specifically, a laminate of a 1 cm $\times$ 1 cm porous polyimide film and a porous polyimide film laminated with a stainless steel mesh (referred to as "liner", not illustrated) having the same area (3 porous polyimide films, 1 liner, 4 porous polyimide films, 1 liner, 3 porous polyimide films, stacked in this order) were sealed in a stainless steel mesh casing to prepare a metal module (FIG. 9 (A)). The operation was performed in a non-sterilized fashion in an open space. A metal drum for operating this metal module was similarly fabricated with a stainless steel mesh (FIG. 9 (B)), and assembled in a non-sterile manner so as to contain 20 metal modules inside. After that, the metal drum containing the metal modules was wrapped with aluminum foil, dry heat sterilized at 190°C for 80 minutes, and allowed to cool.

<Gas phase-exposed type rotation culture apparatus>

Conditioned/suspended anti-human IL-8 antibody producing CHO-DP12 cells (ATCC CRL-12445) were suspension-cultured using a medium (BalanCD (Trademark) CHO GROWTH A) and culture was continued until viable cell count per mL was $1.1 \times 10^6$. As depicted in FIG. 9 (C), a metal drum including a metal module was aseptically placed and prepared ready-for-rotation in a clean environment (FIG. 9 (C)). After 34 mL of the medium obtained by suspension culture of the cells as described above and 6 mL of fresh medium (BalanCD (trademark) CHO GROWTH A) were poured into an upper sump (corresponding to the culture tank 3 in FIG. 1), the metal drum was rotated at a rate of 1 rpm to wet the porous polyimide film with the medium. After leaving the whole apparatus in a $CO_2$ incubator for 21 hours, the medium in the upper sump was removed, and while the rotation of the metal drum was continued, the medium was circulated via a tube pump at a rate of 10 mL/min from a lower sump (corresponding to the medium discharging tank 4 in FIG. 1) in which 200 mL of the medium (KBM-270) was pooled. When culture was performed for 4 days, cells at a cell density of $3.9 \times 10^5$ cells/cm$^2$ with a total cell count of $7.8 \times 10^7$ were observed. After that, the total volume of the medium in the upper and lower sumps was exchanged with a fresh medium (KBM-270), and the culture was continued under the same conditions for another 2 days. At that time, cells at a cell density of $1.3 \times 10^6$ cells/cm$^2$ with a total cell count of $2.6 \times 10^8$ were observed.

REFERENCE SIGNS LIST

1 Cell culture apparatus
2 Cell culture section
20 Porous polymer film
21 Porous polymer film support
22 Porous polymer film clamp
22a Spoke
23 Shaft
24 Clamp ring
25a, 25b Shaft bearing bush
26 Flange
27 Shaft penetrating port
28 Medium passing port
29 Porous polymer film clamp ring
200, 200a, 200b, 200c Cylindrical container
201 End face
202 Side face
203 Hook
204, 205 Medium flow inlet
206 Shaft penetrating port
207 Helical flow path
2000 Stainless steel mesh cylindrical container
2023 Shaft
3 Medium tank
31a, 31b Shaft bearing
310 Shaft bearing depression
32 Medium storage unit
33 Medium discharging pipe
34 Medium supplying pipe
35 Medium storage unit
351 Medium storage unit
36 Medium overflow section
37 Medium discharging pipe
38 Tapered section
4 Medium discharging tank
41 Medium supplying line
42 Medium discharging line
5 Pump
6 Rotary motor
61 Rotary motor shaft
62 Shaft joint section
7 Installation table
8 Modularized porous polymer film
81 Stainless steel mesh

**Claims**

1. A cell culture apparatus, the apparatus **characterized by** comprising:

   a porous polymer film;
   a cell culture section having the porous polymer film;
   a shaft penetrating the cell culture section; and
   a medium tank immersing at least a part of the cell culture section;

   wherein the porous polymer films are a three-layer structure porous polymer film having a surface layer A and a surface layer B, the surface layers having a plurality of pores, and a macrovoid layer sandwiched between the surface layers A and B;
   wherein an average pore diameter of the pores present in the surface layer A is smaller than an average pore diameter of the pores present in the surface layer B;
   wherein the macrovoid layer has a partition wall bonded to the surface layers A and B, and a plurality of macrovoids surrounded by such a partition wall and the surface layers A and B;
   wherein the pores in the surface layers A and B communicate with the macrovoid; and wherein the cell culture section rotates around the shaft, and cells carried on the porous polymer films are alternately cultured in a gas phase and a liquid phase.

2. The cell culture apparatus according to claim 1, the apparatus further comprising a rotary motor for rotating the shaft.

3. The cell culture apparatus according to claim 1 or 2, wherein the cell culture section comprises a porous polymer film support, and a porous polymer film clamp,
   wherein the porous polymer film is sandwiched between the porous polymer film support and the porous polymer film clamp.

4. The cell culture apparatus according to claim 3, wherein two or more sheets of the porous polymer films are stacked and sandwiched.

5. The cell culture apparatus according to claim 1 or 2, wherein the cell culture section is a cylindrical container;
   wherein the end surface of the cylindrical container comprises one or more medium flow inlets;
   wherein the side surface of the cylindrical container comprises one or more medium flow inlets; and
   wherein the porous polymer film is contained in the cylindrical containers.

6. The cell culture apparatus according to claim 5, wherein the cylindrical container comprises one or more helical flow paths that communicate a part of one end face of the cylindrical container with a part of the other end face.

7. The cell culture apparatus according to claim 5 or 6, wherein the porous polymer film is contained in the cylindrical container, with the porous polymer film:

   i) being folded up;
   ii) being wound into a roll-like shape;
   iii) sheets or pieces thereof being concatenated with a thread-like structure;
   iv) being tied together into a rope-like shape; and/or
   v) two or more thereof being stacked.

8. The cell culture apparatus according to claim 5 or 6, wherein the porous polymer film is a modularized porous polymer film having a casing;
   wherein the modularized porous polymer film is contained within the casing with:

   (i) the two or more independent porous polymer films being aggregated;
   (ii) the porous polymer film being folded up;
   (iii) the porous polymer film being wound into a roll-like shape; and/or
   (iv) the porous polymer film being tied together into a rope-like shape;

   wherein the modularized porous polymer film is contained in the cylindrical container.

9. The cell culture apparatus according to any one of claims 1 to 8, wherein two or more of the cell culture sections have been concatenated.

10. The cell culture apparatus according to any one of claims 1 to 9, the apparatus further comprising:

   a medium discharging line communicating with the medium tank at one end;
   a medium discharging tank communicating with the other end of the medium discharging line;
   a medium supplying line communicating at one end with the culture medium discharging tank; and
   a medium supplying pump provided in the middle of the medium supplying line.

11. The cell culture apparatus according to any one of claims 1 to 10, **characterized by** further comprising medium droplet supply means, wherein a dropletized medium is supplied to the porous polymer film.

12. The cell culture apparatus according to any one of claims 1 to 11, wherein the porous polymer film has a plurality of pores having an average pore diameter of 0.01 to 100 $\mu$m.

13. The cell culture apparatus according to any one of claims 1 to 12, wherein an average pore diameter of the surface layer A is 0.01 to 50 $\mu$m.

14. The cell culture apparatus according to any one of claims 1 to 13, wherein an average pore diameter of the surface layer B is 20 to 100 $\mu$m.

15. The cell culture apparatus according to any one of claims 1 to 14, wherein a total film thickness of the porous polymer film is 5 to 500 $\mu$m.

16. The cell culture apparatus according to any one of claims 1 to 15, wherein the porous polymer film is a porous polyimide film.

17. The cell culture apparatus according to claim 16, wherein the porous polyimide film is a porous polyimide film comprising a polyimide derived from tetracarboxylic dianhydride and diamine.

18. The cell culture apparatus according to claim 16 or 17, wherein the porous polyimide film is a colored porous polyimide film that is obtained by molding a polyamic acid solution composition comprising a polyamic acid solution derived from tetracarboxylic dianhydride and diamine, and a coloring precursor, and subsequently heat-treating the resultant composition at 250°C or higher.

19. The cell culture apparatus according to any one of claims 1 to 15, wherein the porous polymer film is a porous polyethersulfone film.

20. A culture method using the cell culture apparatus according to any one of claims 1 to 19, the method **characterized by** comprising:
   rotating the cell culture section around the shaft, and alternately culturing the cell carried on the porous polymer film in a gas phase and a liquid phase.

# FIG. 1

FIG. 2

FIG. 3

(A)

(B)

(C)

(D)

# FIG. 4

(A)

21

21

27          28

(B)

22a          22

22a

29          27

22

29

# FIG. 5

# FIG. 6

MESH SURFACE

LARGE PORE SURFACE

APPARENT MEMBER VOLUME

MEMBER VOLUME CONTAINING CELL SURVIVAL ZONE

# FIG. 7

FIG. 8

# FIG. 9

## (A)

## (B)

## (C)

**INTERNATIONAL SEARCH REPORT**

International application No.

PCT/JP2017/026943

| A. CLASSIFICATION OF SUBJECT MATTER |
| --- |
| *C12M3/00*(2006.01)i, *C12M1/00*(2006.01)i, *C12N1/00*(2006.01)i, *C12N5/00* (2006.01)i |

According to International Patent Classification (IPC) or to both national classification and IPC

| B. FIELDS SEARCHED |
| --- |
| Minimum documentation searched (classification system followed by classification symbols) |
| C12M3/00, C12M1/00, C12N1/00, C12N5/00 |

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS(STN)

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | WO 2015/012415 A1 (Ube Industries, Ltd.), 29 January 2015 (29.01.2015), claims; drawings; examples; paragraphs [0072] to [0101] & US 2016/0168560 A1 claims; figures; examples; paragraphs [0113] to [0145] & EP 3026108 A1 & CN 105452440 A & KR 10-2016-0034303 A | 1-5,7-20 |
| Y | SUCK K et al., A rotating bed system bioreactor enables cultivation of primary osteoblasts on well-characterized Sponceram regarding structural and flow properties, Biotechnol. Prog., 2010, 26(3), p.671-678, Abstract, Figure1 | 1-5,7-20 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 19 September 2017 (19.09.17) | 03 October 2017 (03.10.17) |

| Name and mailing address of the ISA/ Japan Patent Office 3-4-3,Kasumigaseki,Chiyoda-ku, Tokyo 100-8915,Japan | Authorized officer |
| --- | --- |
| | Telephone No. |

Form PCT/ISA/210 (second sheet) (January 2015)

**INTERNATIONAL SEARCH REPORT**

International application No.

PCT/JP2017/026943

C (Continuation).   DOCUMENTS CONSIDERED TO BE RELEVANT

| Category* | Citation of document, with indication, where appropriate, of the relevant passages | Relevant to claim No. |
|---|---|---|
| Y | DIEDERICHS S et al., Dynamic cultivation of human mesenchymal stem cells in a rotating bed bioreactor system based on the ZRP platform, Biotechnol. Prog. 2009, 25(6), p.1762-1771, Abstract,Figures1,2 | 1-5,7-20 |
| Y | JP 55-111785 A  (Makoto SHODA), 28 August 1980 (28.08.1980), claims; drawings; examples (Family: none) | 1-5,7-20 |
| Y | JP 53-14633 B2  (Connaught Laboratories, Ltd.), 18 May 1978 (18.05.1978), claims; drawings; examples & CA 945896 A claims; figures; examples | 1-5,7-20 |
| Y | JP 2002-159288 A  (Nicca Chemical Co., Ltd.), 04 June 2002 (04.06.2002), claims; drawings; examples (Family: none) | 1-5,7-20 |
| Y | JP 5-41984 A  (The Dow Chemical Co.), 23 February 1993 (23.02.1993), claims & US 5162225 A claims & EP 475303 A2 | 19,20 |
| Y | JP 2009-538617 A  (CHA Biotech Co., Ltd.), 12 November 2009 (12.11.2009), claims & US 2009/0130754 A1 claims & EP 2021464 A1         & KR 10-0744445 B1 | 19,20 |
| Y | JP 2012-503688 A  (Gambro Lundia AB.), 09 February 2012 (09.02.2012), claims; examples & US 2011/0263022 A1 claims; examples & EP 2168666 A1       & CN 102202771 A & KR 10-2011-0086006 A | 19,20 |

Form PCT/ISA/210 (continuation of second sheet) (January 2015)

## REFERENCES CITED IN THE DESCRIPTION

**Patent documents cited in the description**

- WO 2003054174 A
- WO 2010038873 A
- JP 2011219585 A
- JP 2011219586 A
- WO 2015012415 A
- WO 2009123349 A
- JP 2009057041 W